# EUROPEAN PATENT APPLICATION

(11) **EP 0 795 556 A1**
(43) Date of publication of application: **17.09.1997**
(21) Application number: 97200386.7
(22) Date of filing: 11.02.1997
(51) Int. Cl.: C07D 487/04, A61K 31/495

(54) **4-Substituted pyrrolopyrimidine compounds as tyrosin kinase inhibitors**

(30) Priority: 29.02.1996 GB 9604361
(71) Applicant: PHARMACIA & UPJOHN S.p.A., 20152 Milano (IT)
(72) Inventor: Buzzetti, Franco, 20052 Monza (Milan) (IT); Brasca, Maria Gabriella, 15 20090 Cusago (Milan) (IT); Longo, Antonio, 20131 Milan (IT); Ballinari, Dario, 20097 San Donato Milanese (Milan) (IT)

(57) **Abstract**

Novel 4-substituted 7H-pyrrolo[2,3-d]pyrimidine derivatives, useful as tyrosine kinase inhibitors, encompassed by following formula (I) 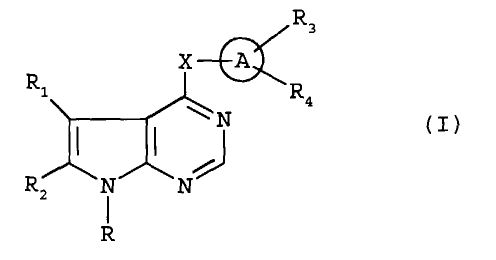 wherein
- X is: -CH₂-, -NH-(CH₂)ₙ-, -O-(CH₂)ₙ- or -S-(CH₂)ₙ- in which n is zero or 1;
- A is: a mono- or bicyclic ring chosen from phenyl, pyridine, tetralin, indan, 2-oxindole, quinoline, isoquinoline and indole;
- R is: hydrogen, C₁-C₄ alkyl or benzyl;
- each of R₁ and R₂,: independently, is hydrogen, halogen, C₁-C₄ alkyl, C₁-C₄ alkoxy, NR₅R₆ in which each of R₅ and R₆ independently is hydrogen or C₁-C₄ alkyl, phenyl unsubstituted or substituted by one to three substituents chosen from halogen, trifluoromethyl, C₁-C₄ alkyl and C₁-C₄ alkoxy;
- each of R₃ and R₄,: independently, is hydrogen, C₁-C₄ alkyl, halogen, hydroxy, C₁-C₄ alkoxy, C₁-C₄ alkoxycarbonyl, nitro, cyano or trifluoromethyl;
and the pharmaceutically acceptable salts thereof are provided.

## Description

The present invention relates to new 4-substituted 7H-pyrrolo[2,3-d]pyrimidine compounds, to a process for their preparation, to pharmaceutical compositions containing them and to their use as therapeutic agents, in particular as tyrosine kinase inhibitors.
Australian patent application 9222817 discloses 4-benzylamino substituted pyrrolo[2,3-d]pyrimidines having hair growth promoting activity. EP-A-478292 describes 4-benzyloxy substituted pyrrolo[2,3-d]pyrimidines useful as intermediate compounds in the preparation of purine nucleoside phosphorilase inhibitors. J.Pharm.Sci. 54(12), 1826-7 (1965) describes the preparation of 4-benzylthio-7H-pyrrolo[2,3-d] pyrimidine. The preparation of several 4-substituted pyrrolo[2,3-d]pyrimidines is also disclosed in J.Heterocyclic Chem. 22, 859 (1985) and ibidem 6(2), 207-13 (1969).

The present invention provides novel 4-substituted 7H-pyrrolo[2,3-d]pyrimidine compounds having the following formula (I) 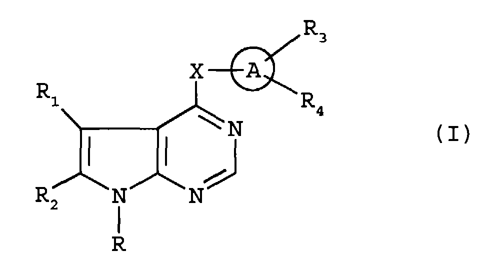 wherein
- X is: -CH₂-, -NH-(CH₂)ₙ, -O-(CH₂)ₙ- or -S-(CH₂)ₙ- in which n is zero or 1;
- A is: a mono- or bicyclic ring chosen from phenyl, pyridine, tetralin, indan, 2-oxindole, quinoline, isoquinoline and indole;
- R is: hydrogen, C₁-C₄ alkyl or benzyl;
- each of R₁ and R₂,: independently is chosen from hydrogen, halogen, C₁-C₄ alkyl, C₁-C₄ alkoxy, NR₅R₆ in which each of R₅ and R₆ independently is hydrogen or C₁-C₄ alkyl and phenyl unsubstituted or substituted by one to three substituents chosen from halogen, trifluoromethyl, C₁-C₄ alkyl and C₁-C₄ alkoxy;
- each of R₃ and R₄,: independently, is hydrogen, C₁-C₄ alkyl, halogen, hydroxy, C₁-C₄ alkoxy, C₁-C₄ alkoxycarbonyl, nitro, cyano or trifluoromethyl;
and the pharmaceutically acceptable salts thereof;
and wherein, when at the same time, X is -NH-(CH₂)ₙ-, -O-(CH₂)ₙ- or -S-(CH₂)ₙ-, R is hydrogen or C₁-C₄ alkyl and A is phenyl, pyridine, quinoline, isoquinoline or indole, then at least one of R₁ and R₂ is other than hydrogen, halogen, C₁-C₄ alkyl, C₁-C₄ alkoxy or NR₅R₆ as defined above;
and wherein, when at the same time, X is -NH-, A is phenyl; one of R₁ and R₂ is hydrogen, C₁-C₄ alkyl or phenyl unsubstituted or substituted by halogen, trifluoromethyl, C₁-C₄ alkyl or C₁-C₄ alkoxy and the other is C₁-C₄ alkyl or phenyl unsubstituted or substituted by halogen, trifluoromethyl, C₁-C₄ alkyl or C₁-C₄ alkoxy; and R₃ and R₄ are halogen, trifluoromethyl, C₁-C₄ alkyl or C₁-C₄ alkoxy;
then R is other than hydrogen.
The X bridge may be located on either of the ring A moieties when A is a bicyclic ring, preferably it is located on the benzene moiety. The R₃ and R₄ substituents in tetralin, indane, quinoline, isoquinoline and indole may be on either of the ring moieties, preferably they are attached to the benzene moiety. In 2-oxindole the R₃ and R₄ substituents are only located on the benzene moiety.
The invention includes within its scope all the possible isomers, stereoisomers and their mixtures, and the metabolites and the metabolic precursors or bio-precursors (otherwise known as prodrugs) of the compounds of formula (I).
The X bridge is preferably linked to position 1 or 2 when A is tetralin, to position 5 when A is indane, indole or 2-oxindole, to position 5 or 6 when A is quinoline or isoquinoline.
Of course only one of the X, R₃ and R₄ substituents can be linked to the same position in ring A.
An alkyl group or an alkyl moiety in a alkoxy group may be branched or straight alkyl chain.
A C₁-C₄ alkyl group is preferably a C₁-C₂ alkyl, that is ethyl or methyl.
A C₁-C₄ alkoxy group is preferably a methoxy or ethoxy group.
A halogen atom is for example fluoro, chloro, bromo or iodio, in particular bromo or fluoro.
The term tetralin is meant to refer to 5,6,7,8-tetrahydro-naphthalene.
In term X when X is -NHCH₂-,-OCH₂- or -SCH₂- it is understood that the linkage with the pyrrolo[2,3-d]pyrimidine ring occurs through the N, O or S atom.
Pharmaceutically acceptable salts of the compounds of the invention include acid addition salts with inorganic acids, e.g. nitric, hydrochloric, hydrobromic, sulphuric, perchloric and phosphoric acid or organic acids, e.g. acetic, trifluoracetic, propionic, glycolic, lactic, oxalic, malonic, malic, maleic, tartaric, citric, benzoic, cinnamic, mandelic and salicylic acid.
As stated above, the present invention also includes within its scope pharmaceutically acceptable bio-precursors (otherwise known as prodrugs of the compounds of formula (I), i.e. compounds which have different formula to formula (I) above but which, nevertheless, upon administration to a human being are converted directly or indirectly in vivo into a compound of formula (I).

Preferred compounds of the invention are the compounds of formula (I), wherein
X is -O-, -S-, -CH₂- or -NH-(CH₂)ₙ- in which n is zero or 1;
A is tetralin, indan or 2-oxindole;
R is hydrogen;
R₁ and R₂ are H;
R₃ and R₄ are H or halogen;
and the pharmaceutically acceptable salts thereof.

Examples of preferred specific compounds of formula (I) are the following compounds:
4-(2-tetralylthio)-7H-pyrrolo[2,3-d]pyrimidine;
4-(5-indanylamino)-7H-pyrrolo[2,3-d]pyrimidine;
4-(4-indanylamino)-7H-pyrrolo[2,3-d]pyrimidine;
4-(1-tetralylamino)-7H-pyrrolo[2,3-d]pyrimidine;
4-(2-tetralylamino)-7H-pyrrolo[2,3-d]pyrimidine;
4-(5-tetralylamino)-7H-pyrrolo[2,3-d]pyrimidine;
4-(2-oxindol-5-ylamino)-7H-pyrrolo[2,3-d]pyrimidine;
4-(7-bromo-5-indanylamino)-7H-pyrrolo[2,3-d]pyrimidine;
4-(6-bromo-4-indanylamino)-7H-pyrrolo[2,3-d]pyrimidine; 4-(3-bromo-1-tetralylamino)-7H-pyrrolo[2,3-d]pyrimidine;
4-(4-bromo-2-tetralylamino)-7H-pyrrolo[2,3-d]pyrimidine;
4-(5-indanyloxy)-7H-pyrrolo[2,3-d]pyrimidine;
4-(1-tetralyloxy)-7H-pyrrolo[2,3-d]pyrimidine;
4-(2-tetralyloxy)-7H-pyrrolo[2,3-d]pyrimidine;
4-(2-oxindol-5-yloxy)-7H-pyrrolo[2,3-d]pyrimidine;
4-(5-indanylmethyl)-7H-pyrrolo[2,3-d]pyrimidine;
4-(2-tetralylmethyl)-7H-pyrrolo[2,3-d]pyrimidine;
4-(2-oxindol-5-ylmethyl)-7H-pyrrolo[2,3-d]pyrimidine;
4-benzyl-7H-pyrrolo[2,3-d]pyrimidine;
4-(4-pyridylmethyl)-7H-pyrrolo[2,3-d]pyrimidine;
4-(5-indolylmethyl)-7H-pyrrolo[2,3-d]pyrimidine; and
4-(6-quinolylmethyl)-7H-pyrrolo[2,3-d]pyrimidine;
either as single isomers or as a mixture thereof and the pharmaceutically acceptable salts thereof.

A further object of the present invention is also to provide a 4-substituted pyrrolo[2,3-d]pyrimidine compound of formula (I) as defined above, or a pharmaceutically acceptable salt thereof, for use as an active therapeutic substance, in particular as tyrosine kinase inhibitor.
Object of the invention is also a pharmaceutical composition comprising a compound of formula (I), or a pharmaceutically salt thereof, as defined above, as an active principle and a pharmaceutically acceptable excipient (which can be a carrier and/or diluent).
The compounds of formula (I) and the pharmaceutically acceptable salts thereof, are defined hereafter as the "compounds of the invention" or as the "active agents" of the invention.

The compounds of formula (I), and the salts thereof, can be obtained by a process comprising:
a) reacting a compound of formula (II) 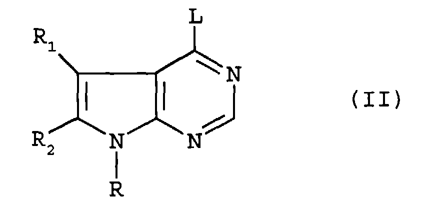 wherein R, R₁ and R₂ are as defined above and L is a leaving group, with an amine compound of formula (III) 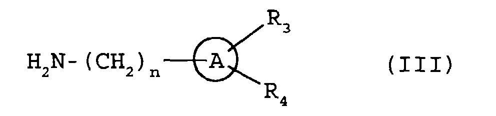 wherein n, A, R₃ and R₄ are as defined above, thus obtaining a compound of formula (I) in which X is -NH-(CH₂)ₙ-; or
b) reacting a compound of formula (II) as defined above, with an hydroxy compound of formula (IV) 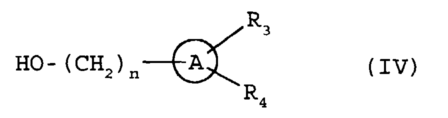 wherein n, A, R₃ and R₄ are as defined above, thus obtaining a compound of formula (I) in which X is -O-(CH₂)ₙ-; or
c) reacting a compound of formula (II) as defined above, with a thio compound of formula (V) 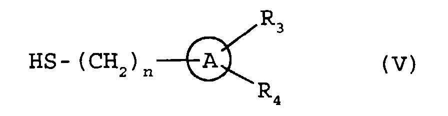 wherein n, A, R₃ and R₄ are as defined above, thus giving a compound of formula (I) in which X is -S-(CH₂)ₙ-; or
d) hydrolyzing and decarboxylating a compound of formula (VI) 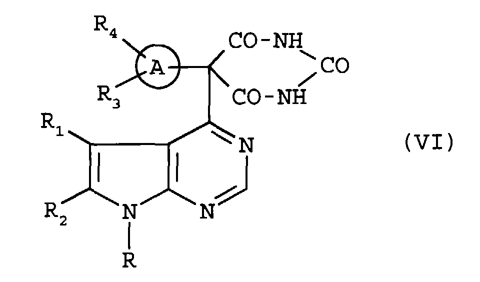 wherein A, R, R₁, R₂, R₃ and R₄ are as defined above, thus providing compounds of formula (I), wherein X is -CH₂-;
and, if desired, converting a compound of formula (I) into another compound of formula (I), and/or, if desired, converting a compound of formula (I) into a salt thereof, and/or, if desired, converting a salt of a compound of formula (I) into a free compound of formula (I), and/or, if desired, separating a mixture of isomers of a compound of formula (I) into the single isomers.
A leaving group L in a compound of formula (II) is for instance chloro, 1,2,4-triazol-1-yl or methylthio.
The reaction of a compound of formula (II) wherein L is chloro with a compound of formula (III) according to process step a) may be carried out using known methods, e.g. as described by Bullock et al. in J.Am.Chem.Soc. 78, 3693 (1956). The reaction is carried out in the presence of a suitable organic inert solvent, for example an alkanol or ester such as methanol, ethanol, isopropanol, methyl cellosolve or ethyl acetate, a halogenated solvent such as dichloromethane or chloroform, an ether such as tetrahydrofuran or dioxane, a dipolar aprotic solvent such as dimethyl formamide or dimethyl acetamide. Preferably the solvents isopropanol or methyl cellosolve are used. The reaction is conveniently carried out at a temperature in the range from about 10 to about 150°C, preferably in the range from about 20 to about 80°C. In general only 1 equivalent of amine compound (III) is used, thus giving the hydrochloride salt, which precipitates on cooling. To obtain the free base from the salt, the salt may be treated with a suitable base in the presence of an appropriate solvent such as the ones mentioned above. Suitable bases are e.g. organic amines such as triethylamine or pyridine, or inorganic bases such as sodium carbonate or sodium hydroxide. Alternatively to obtain directly the free base of formula (I) one may apply more than 2 equivalent of amine compound (III) in the reaction.
The reaction of a compound of formula (II) wherein L is chloro with a compound of formula (IV) according to process step b) may be carried out by using known methods, e.g. as described by Prasad et al. in J.Am.Chem.Soc. 79, 6401 (1957). The reaction is preferably carried out in a protic solvent, e.g. water or aqueous alkanol such as aqueous methanol, ethanol or isopropanol in the presence of a suitable alkali base such as sodium or potassium hydroxide. The reaction temperatures are ranging from about 0 to about 100°C, preferably the range is from about 50 to about 100°C. Alternatively the hydroxy compound of formula (IV) is at first transformed into its metal salt in an aprotic solvent, which is then reacted with the compound of formula (II). For example the metallation of compound (IV) may be carried out with metal compounds like NaH or NaNH₂ in an aprotic solvent such as tetrahydrofuran, ethyl ether, DMF or benzene. The metal salt is then reacted with compound (II) in the same aprotic solvent at temperatures ranging from about 0 to about 100°C, preferably in the range from about 20 to about 40°C.
The reaction of a compound of formula (II) with a thiol compound of formula (V) according to process step c) may be carried out using known methods, e.g. as reviewed in Heterocyclic Compounds vol.8, page 335 (1967, Editor R.C. Elderfield). Suitable reaction solvents are protic solvents, e.g. water, alkanols such as methanol, ethanol and isopropanol or ethers such as tetrahydrofuran or dioxane. In order to obtain the corresponding metal mercaptide, which is the actual reactant, the reaction is carried out in the presence of a suitable alkali base, e.g. an alkali hydroxide such as sodium or potassium hydroxide, an alkali alkoxide such as sodium or potassium methoxide, sodium or potassium ethoxide or sodium or potassium methoxyethoxide. The reaction temperature may vary from about 0 to about 120°C, preferably from 40 to 80°C.
The hydrolysis and decarboxylation of a compound of formula (VI) according to process step d) may be carried out using known methods, e.g. as described in J.Het.Chem. 14, 1081 (1977) by A. Scoville and F.X.Smith. Suitable reaction solvents are protic solvents, e.g. water or aqueous alkanols such as methanol, ethanol or isopropanol. The hydrolysis step is carried out in alkaline conditions, e.g. in the presence of an alkali hydroxide such as sodium or potassium hydroxide. The reaction temperature may range from room to reflux temperature, preferably reflux temperature is applied. The decarboxylation step is carried out in slightly acidic conditions, e.g. in the presence of a mineral acid such as hydrochloric acid. The reaction temperature may vary from room to reflux temperature, preferably reflux temperature is used.
The optional salification of a compound of formula (I) as well as the conversion of the salt into the corresponding free compound and the separation of the mixture of isomers into the single isomers as well as the conversion of a compound of formula (I) into another compound of formula (I) may be carried according to known methods.

The conversion of a compound of formula (I), wherein X is -O-(CH₂)ₙ- or -S-(CH₂)ₙ- into a compound (I), wherein X is -NH-(CH₂)ₙ- can be carried out, according known methods, by a displacement reaction with an amine compound of formula (III) as defined above. E.g. according to Elion et al. in J.Am.Chem.Soc. 74, 411 (1952) the thio compound of formula (I) is heated with the amine compound of formula (III) in aqueous solution in a sealed tube at temperatures ranging from about 130 to about 180°C.

The compounds of formula (II) are known or may be obtained by known methods from known compounds. For example the 4-chloro compounds of formula (II) wherein L is chloro are prepared by chlorodehydroxylation of the corresponding 4-hydroxy-7H-pyrrolo[2,3-d]pyrimidine derivatives of formula (VII) 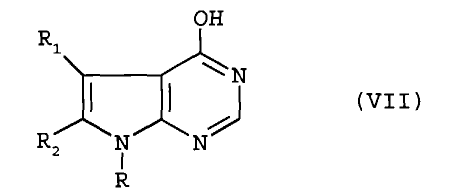 using conventional methods, e.g. by reaction with POCl₃. The intermediate of formula (II), wherein L is 1,2,4-triazol-1-yl, can be prepared e.g. by adding gradually POCl₃ to a mixture of compound of formula (VII) (1 equivalent) and 1,2,4-triazole (3 equivalent) in pyridine solution at temperatures ranging from room to reflux temperature.
The compounds of formula (VII) are known or may be obtained by known methods from known compounds. For example 4-hydroxy-7H-pyrrolo[2,3-d]pyrimidine is obtained by Raney-Nickel reduction of 4-hydroxy-2-mercapto-7H-pyrrolo[2,3-d] pyrimidine, which in turn is obtained by alkaline condensation of 4,4-diethoxy-2-cyanobutyric acid ethyl ester and thiourea as described in Chem.Ber. 111, 2925 (1978). 6-Phenyl-4-hydroxy-7H-pyrrolo[2,3-d]pyrimidine may be obtained from 2-amino-3-carbethoxy-5-phenylpyrrole by condensation with formamide. The latter compound is prepared by alkaline condensation (NaOEt) of phenacylbromide with ethyl amidinoacetate.

The compounds of formula (VI) can be made by using the process of A.Scoville and F.X.Smith as described in J.Het.Chem. 14, 1081 (1977). Accordingly a compound of formula (II), in which L is chloro and R₁ and R₂ are as defined above, is reacted with a compound of formula (VIII) 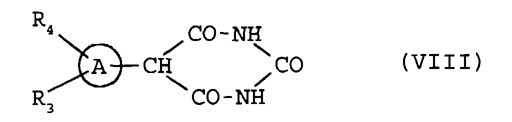 in which A, R₃ and R₄ are as defined above.
The compounds of formulae (III), (IV), (V) and (VIII) are either known compounds or may be obtained by known methods from known compounds.
When in the new compounds of the present invention and in the intermediate products used for their preparation there are groups present which need to be protected before the above-described reactions are performed, they may be protected before the reaction takes place and then deprotected at the end of the reaction, according to well known methods in organic chemistry.

### PHARMACOLOGY

The compounds of the invention possess specific tyrosine kinase inhibiting activity. It is believed that tyrosine kinase inhibitors may be of great importance in the control of uncontrolled cellular reproduction, i.e. in cellular reproduction disorders. Hence, the compounds according to the present invention can be useful in the treatment of pathological proliferation disorders in mammals, including humans. Typical examples of such disorders are tumors, including leukemia, and psoriasis. The compounds of the invention can also be useful in inhibiting the development of the atheromatous plaque and in the control of angiogenesis and as anti-metastatic agents.
Recent studies on the molecular basis of the neoplastic transformation have identified a family of genes, designed oncogenes, whose aberrant expression causes tumorigenesis. For example, the RNA tumor viruses possess such an oncogene sequence whose expression determines neoplastic conversion of infected cells. Several of their oncogene-encoded proteins, such as pp60^{v-src}, p70^{gag-yes}, p130^{gag-fps} and p70^{gag-fgr} display protein tyrosine kinase activity, that is they catalyze the transfer of the γ-phosphate from adenosine triphosphate (ATP) to tyrosine residues in protein substrate. In normal cells, several growth factor receptors, for example the receptors for PDGF, EGF, α-TGF and insulin, display tyrosine kinase activity. Binding of the growth factor (GF) activates the receptor tyrosine kinase to undergo autophosphorylation and to phosphorylate closely adjacent molecules on tyrosine. Therefore, it is thought that the phosphorylation of these tyrosine kinase receptors plays an important role in signal transduction and the principal function of tyrosine kinase activity in normal cells is to regulate cell growth. Perturbation of this activity by oncogenic tyrosine kinases that are either overproduced and/or display altered substrate specificity may cause loss of growth control and/or neoplastic transformation. Accordingly, a specific inhibitor of tyrosine kinase can be useful in investigating the mechanism of cancerogenesis, cell proliferation and differentiation and it can be effective in the prevention and chemotherapy of cancer and in other pathological proliferative conditions.
Hence the compounds according to the present invention can be useful in the treatment of pathological proliferation disorders in mammals, including humans.
A human or animal, e.g. a mammal, can thus be treated by a method comprising the administration thereto of therapeutically effective amount of one of the compounds of the invention. In this way the condition of the human or animal may be improved. Amelioration of the disease state or disorder from which the human or animal is suffering can be achieved. Typical examples of such disorders are benign and malignant tumours, including leukaemia such as myeloblastic leukaemia, lymphoma, sarcoma, neuroblastoma, Wilm's tumour, malignant neoplasm of the bladder, breast, lung or thyroid, neoplasias of epithelial origin, such as mammacarcinoma. Moreover, they can be useful in the treatment of epidermal hyperprolifertion, such as psoriasis. The compounds of the invention can also be useful in inhibiting the development of the atheromatous plaque and restenosis, in the control of angiogenesis, as anti-metastatic agents and in treating diabetic complications. They have also utility in the control of immune system diseases, e.g. as immunosuppressants, as far as protein tyrosine kinases are involved in these diseases.
The tyrosine specific protein kinase activity of the compounds of the invention is shown, e.g., by the fact that they are active in the in vitro and in vivo test described herebelow.

### In Vitro Assay

### p45 v-abl kinase purification

The enzyme used in our tests is the p45 v-abl tyrosine kinase which represents the catalytic domain of the Abelson tyrosine kinase (isolated from the Abelson murine leukemia virus). The p45 v-abl kinase is produced and isolated as described by Wang et al. in J.Biol.Chem. 260, 64 (1985) and by Ferguson et al. in J.Biol.Chem. 260, 3652 (1985) and in Biochem.J. 257, 321 (1989).

### p45 v-abl kinase assay

(Val⁵)-angiotensin II phosphorylation is performed by incubation with 40 ng of purified abl-kinase and (γ-³²P) ATP, in 50 µl of buffer containing Tris-HCl 25 mM, pH 8.0, MgCl₂ 10 mM and dithiothreitol 0.1 mM (kinase buffer). The reaction mixture is incubated for the indicated time at 30°C and the reaction stopped by adding 50 µl of 5% trichloracetic acid. After a brief incubation on ice the tubes are centrifuged. The supernatants are spotted on phosphocellulose paper squares (Whatman P-81) and washed extensively in acetic acid. The radioactivity bound to dried phosphocellulose squares is measured in a liquid scintillation counter. IC₅₀ values are calculated from triplicated determination of each experimental point. Each inhibitor is tested at concentrations ranging from 0 to 400 µg in the presence of fixed concentrations of peptide (2 mM) and ATP (50 µM).

### In Vivo Assay

### K562 cell growth inhibition assay

K562 cells, a human myelogenous leukemia cell line, were seeded into a 24 wells tissue culture plate (Falcon 3047) (10000/well) in the presence of increasing concentrations of the compounds. After 72 h, cells were harvested and were counted using a cell counter (Coulter Counter-ZM). The percent of inhibition was evaluated in respect to the untreated control cells.
In view of their high activity and low toxicity, the compounds of the invention can be used safely in medicine.
The compounds of the invention can be administered in a variety of dosage forms, e.g. orally, in the forms of tablets, capsules, sugar- and film-coated tablets, liquid solutions or suspensions; rectally, in the form of suppositories; parenterally, e.g. intramuscularly, or by intravenous injection or infusion; or topically. The dosage depends on the age, weight, condition of the patient and administration route. For example, the dosage adopted for oral administration to adult humans for the compound 4-(5-indanylamino)-7H-pyrrolo[2,3-d]pyrimidine may range from about 5 to about 150-200 mg per dose, from 1 to 5 times daily. Of course, these dosage regimes may be adjusted to provide the optimal therapeutic response.
The pharmaceutical compositions containing the compounds of the invention are usually prepared following conventional methods and are administered in a pharmaceutically suitable form.
For example, the solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents, e.g. starches, arabic gums, gelatin, methylcellulose, carboxymethylcellulose or polyvinyl pyrrolidone; disaggregating agents, e.g. a starch, alginic acid, alginates or sodium starch glycolate, effervescing mixtures; dyestuffs; sweeteners; wetting agents, such as lecithin, polysorbates, laurylsulphates; and, in general, non-toxic and pharmacologically inactive substances used in pharmaceutical formulations. Said pharmaceutical preparations may be manufactured in known manner, by means of mixing, granulating, tabletting, sugar-coating or film-coating processes.
The liquid dispersion for oral administration may be, e.g., syrups, emulsions and suspensions.
The syrup may contain as carrier, for example, saccharose or saccharose with glycerine and/or mannitol and/or sorbitol.
The suspensions and the emulsions may contain as carrier, for example, a natural gum, agar, sodium alginate, pectin, methylcellulose, carboxymethylcellulose or polyvinyl alcohol. The suspensions or solutions for intramuscular injections may contain, together with the active compound, a pharmaceutically acceptable carrier, e.g. sterile water, olive oil, ethyl oleate, glycols, e.g. propylene glycol, and, if desired, a suitable amount of lidocaine hydrochloride.
The solutions for intravenous injections or infusions may contain as carrier, for example, sterile water or, preferably, they may be in the form of sterile aqueous, isotonic saline solutions.
The suppositories may contain, together with the active compound, a pharmaceutically acceptable carrier, e.g. cocoa-butter, polyethylene glycol, a polyoxyethylene sorbitan fatty acid ester surfactant or lecithin.
Compositions for topical application, e.g. creams, lotions or pastes, can be prepared by admixing the active ingredient with a conventional oleaginous or emulsifying excipient.
A further object of the present invention is a combined method of treatment of cancer or of amelioration of the conditions of mammals, including humans, suffering from cancer, said method comprising administering
1) a compound of the invention, that is a compound of formula (I), or a pharmaceutically acceptable salt thereof, and
2) an additional antitumor agent, in amounts and close enough together in time sufficient to produce a therapeutically useful effect.
The present invention also provides products containing a compound of the invention, that is a compound of formula (I) or a pharmaceutically acceptable salt thereof, and an additional antitumor agent as a combined preparation for simultaneous, separate or sequential use in anticancer therapy.
The term "antitumour agent" is meant to comprise both a single antitumour drug and "cocktails", i.e. a mixture of such drugs, according to the clinical practice.
Examples of antitumour agents that can be formulated with a compound of the invention or, alternatively, can be administered in a combined method of treatment, include doxorubicin, daunomycin, epirubicin, idarubicin, etoposide, fluorouracil, melphalan, cyclophosphamide, bleomycin, vinblastin and mitomycin or a mixture of two or more thereof. The compounds of the invention can therefore be used in a treatment to ameliorate a cancer. They may be administered to a patient suffering from a cancer treatable with an antitumour agent, for example an anthracycline glycoside such as doxorubicin, daunomycin, epirubicin or idarubicin as mentioned above, together with the antitumour agent.
A compound of the invention and an antitumour agent such as an anthracycline glycoside can be administered to improve the condition of a patient having leukemia such as myeloblastic leukemia, lymphoma, sarcoma, neuroblastoma, Wilm's tumour or malignant neoplasm of the bladder, breast, lung or thyroid.
Accordingly, the present invention provides a method of treating a patient in need of a tyrosine kinase inhibitor, the method comprising administering to said patient a therapeutically effective amount of a compound of formula (I) as defined above, or a pharmaceutically acceptable salt thereof.
The following examples illustrate but do not limit the invention.

### Example 1

### 4-(5-indanylamino)-7H-pyrrolo[2,3-d]pyrimidine hydrochloride.

A solution of 4-chloropyrrolo[2,3-d]pyrimidine (1.536 g, 10 mM) and 5-aminoindan (1.332 g, 10 mM) in isopropanol (60 ml) is heated to reflux for about 20 h. The resulting salt suspension is then cooled to room temperature, filtered and the residue washed with ice-cooled isopropanol to give almost pure title compound in about 75% yield.

| | | | | | |
|---|---|---|---|---|---|
| C₁₅H₁₅ClN₄ | calcd: | C62.83 | H5.27 | C112.36 | N19.54 |
| | found: | C62.45 | H5.15 | Cl12.21 | N19.35 |

MS m/z 286.

According to the above described procedure the following compounds can be prepared:
4-(4-indanylamino)-7H-pyrrolo[2,3-d]pyrimidine hydrochloride;
4-(1-tetralylamino)-7H-pyrrolo[2,3-d]pyrimidine hydrochloride;
4-(2-tetralylamino)-7H-pyrrolo[2,3-d]pyrimidine hydrochloride;
4-(5-tetralylamino)-7H-pyrrolo[2,3-d]pyrimidine hydrochloride;
4-(2-oxindol-5-ylamino)-7H-pyrrolo[2,3-d]pyrimidine hydrochloride;
4-(7-bromo-5-indanylamino)-7H-pyrrolo[2,3-d]pyrimidine hydrochloride;
4-(6-bromo-4-indanylamino)-7H-pyrrolo[2,3-d]pyrimidine hydrochloride;
4-(3-bromo-1-tetralylamino)-7H-pyrrolo[2,3-d]pyrimidine hydrochloride; and
4-(4-bromo-2-tetralylamino)-7H-pyrrolo[2,3-d]pyrimidine hydrochloride.

### Example 2

### 4-(5-indanylamino)-7H-pyrrolo[2,3-d]pyrimidine.

A suspension of 4-(5-indanylamino)-7H-pyrrolo[2,3-d] pyrimidine hydrochloride (2.868 g, 10 mM) and potassium carbonate (2.764 g, 20 mM) in methanol (60 ml) is stirred at ambient temperature for 0.5 h. The mixture is filtered and the filtrate evaporated under vacuum. The residue is purified by column chromatography using a 93:7 mixture of dichloromethane/methanol as eluant to give pure title compound in 85% yield.

| | | | | |
|---|---|---|---|---|
| C₁₅H₁₄N₄ | calcd: | C71.98 | H5.64 | N22.38 |
| | found: | C71.56 | H5.45 | N22.25 |

MS m/z 250.

By proceeding analogously the following compounds can be prepared:
4-(4-indanylamino)-7H-pyrrolo[2,3-d]pyrimidine;
4-(1-tetralylamino)-7H-pyrrolo[2,3-d]pyrimidine;
4-(2-tetralylamino)-7H-pyrrolo[2,3-d]pyrimidine;
4-(5-tetralylamino)-7H-pyrrolo[2,3-d]pyrimidine;
4-(2-oxindol-5-ylamino)-7H-pyrrolo[2,3-d]pyrimidine;
4-(7-bromo-5-indanylamino)-7H-pyrrolo[2,3-d]pyrimidine;
4-(6-bromo-4-indanylamino)-7H-pyrrolo[2,3-d]pyrimidine;
4-(3-bromo-1-tetralylamino)-7H-pyrrolo[2,3-d]pyrimidine; and
4-(4-bromo-2-tetralylamino)-7H-pyrrolo[2,3-d]pyrimidine.

### Example 3

### 4-(2-tetralyloxy)-7H-pyrrolo[2,3-d]pyrimidine.

To a solution of 2-hydroxytetralin (1.482 g, 10 mM) in 80 ml of aqueous potassium hydroxide solution containing 1.683 g (30 mM) of solid potassium hydroxide is added 4-chloro-pyrrolo[2,3-d]pyrimidine (1.536 g, 10 mM). The reaction mixture is stirred for about 0.5 h at room temperature until most of the 6-chloro-pyrido[2,3-d]pyrimidine dissolves and then heated on the steam bath for further 0.5 h. The mixture is cooled, filtered and the residue recrystallized from hot aqueous ethanol to give pure title compound in 65% yield.

| | | | | |
|---|---|---|---|---|
| C₁₆H₁₅N₃O | calcd: | C72.43 | H5.70 | N15.84 |
| | found: | C72.25 | H5.65 | N15.80 |

MS m/z 265.

According to the above described procedure the following compounds can be prepared:
4-(5-indanyloxy)-7H-pyrrolo[2,3-d]pyrimidine;
4-(1-tetralyloxy)-7H-pyrrolo[2,3-d]pyrimidine; and
4-(2-oxindol-5-yloxy)-7H-pyrrolo[2,3-d]pyrimidine.

### Example 4

### 4-(2-tetralylthio)-7H-pyrrolo[2,3-d]pyrimidine.

To a solution of 4-chloropyrrolo[2,3-d]pyrimidine (1.536 g, 10 mM) in methanol (30 ml) is added a solution of 2-mercaptotetralin (4.928 g, 30 mM) in methanolic potassium hydroxide (60 ml containing 1.608g (30 mM) solid potassium hydroxide). The reaction mixture is stirred for 0.5 h at room temperature and then boiled for 0.5 h at reflux. The solution is concentrated under vacuum and then cooled to give crystalline title compound in about 65% yield.

| | | | | | |
|---|---|---|---|---|---|
| C₁₆H₁₅N₃S | calcd: | C68.30 | H5.37 | N14.93 | S11.39 |
| | found: | C68.15 | H5.25 | N14.75 | S11.31 |

MS m/z 281.

### Example 5

### 4-(5-indanylmethyl)-7H-pyrrolo[2,3-d]pyrimidine.

A solution of 5-(5-indanyl)-5-(pyrrolo[2,3-d]pyrimidin-4-yl) barbituric acid (3.614 g, 10 mM) and sodium hydroxide (2.00 g, 50 mM) in water (40 ml) is refluxed for 15 h. After cooling the solution is made slightly acidic (pH4-5) by addition of HCl and again refluxed for 15 h. The solution is cooled, made strongly basic with sodium hydroxide and then extracted with ethyl acetate. The organic phase is washed with water, dried and then evaporated to dryness under vacuum. The residue is purified by column chromatography using dichloromethane/methanol 93:7 as eluant. Thus pure title compound is obtained in about 65% yield.

| | | | | |
|---|---|---|---|---|
| C₁₆H₁₅N₃ | calcd: | C77.08 | H6.06 | N16.85 |
| | found: | C77.01 | H5.95 | N16.75 |

By proceeding analogously the following compounds can be prepared:
4-(2-tetralylmethyl)-7H-pyrrolo[2,3-d]pyrimidine;
4-(2-oxindol-5-ylmethyl)-7H-pyrrolo[2,3-d]pyrimidine;
4-benzyl-7H-pyrrolo[2,3-d]pyrimidine;
4-(4-pyridylmethyl)-7H-pyrrolo[2,3-d]pyrimidine;
4-(5-indolylmethyl)-7H-pyrrolo[2,3-d]pyrimidine; and
4-(6-quinolylmethyl)-7H-pyrrolo[2,3-d]pyrimidine.

### Example 6

### 4-(5-indanylamino)-7H-pyrrolo[2,3-d]pyrimidine.

A suspension of 4-(2-tetralylthio)-7H-pyrrolo[2,3-d] pyrimidine (2.814 g, 10 mM) and 5-aminoindan (3.996 g, 30 mM) in water (100 ml) is heated in a sealed tube at 130°C for 20 h. Then the water is evaporated under vacuum and the residue chromatographed on silica gel by using dichloromethane/ethanol mixtures as eluant. Thus almost pure title compound is obtained in about 45% yield.

| | | | | |
|---|---|---|---|---|
| C₁₅H₁₄N₄ | calcd: | C71.98 | H5.64 | N22.38 |
| | found: | C71.56 | H5.55 | N22.15 |

MS m/z 250.

### Example 7

### 5-(5-indanyl)-5-(7H-pyrrolo[2,3-d]pyrimidin-4-yl)barbituric acid.

A slurry of 4-chloro-7H-pyrrolo[2,3-d]pyrimidine (1.536 g, 10 mM) and 5-(5-indanyl) barbituric acid (2.443 g, 10 mM) is stirred in an oil bath. The temperature is raised to 130°C in a period of 15 min. Then the temperature is further increased from 130°C to 170°C. During this period apparently a reaction occurs since the slurry solidifies. The resulting solid is maintained for further 10 min at about 170°C. Then the reaction mixture is cooled, triturated with sodium bicarbonate solution and hexane. The solid is filtered off, washed with water and dried under vacuum. The raw product is submitted to the next step without further purification.

### Example 8

### 4-chloro-7H-pyrrolo[2,3-d]pyrimidine.

A mixture of 7H-pyrrolo[2,3-d]pyrimidin-4(3H)-one (1.351 g, 10 mM) and POCl₃ (16 ml) is stirred for 1 h at reflux. The excess of POCl₃ is removed under vacuum. Then dichloromethane and iced water is added and the mixture stirred until the black solid dissolves. The organic layer is separated, washed with bicarbonate solution, dried over Na₂SO₄ and then evaporated to dryness. The yellow residue is recrystallized from toluene/hexane to give almost pure title compound in 65% yield; m.p. 188-190°C.

### Example 9

Tablets each weighing 0.150 g and containing 25 mg of the active substance, can be manufactured as follows:

### Composition (for 10,000 tablets):

| | |
|---|---|
| 4-(5-indanylamino)-7H-pyrrolo[2,3-d]pyrimidine | 250 g |
| Lactose | 800 g |
| Corn starch | 415 g |
| Talc powder | 30 g |
| Magnesium stearate | 5 g |

The 4-(5-indanylamino)-7H-pyrrolo[2,3-d]pyrimidine, the lactose and half of the corn starch are mixed; the mixture is then forced through a sieve of 0.5 mm mesh size. Corn starch (10 g) is suspended in warm water (90 ml) and the resulting paste is used to granulate the powder. The granulate is dried, comminuted on a sieve of 1.4 mm mesh size, then the remaining quantity of starch, talc and magnesium stearate is added, carefully mixed and processed into tablets.

### Example 10

Capsules, each dosed at 0.200 g and containing 20 mg of the active substance can be prepared.

### Composition for 500 capsules:

| | |
|---|---|
| 4-(2-tetralylamino)-7H-pyrrolo[2,3-d]pyrimidine | 10 g |
| Lactose | 80 g |
| Corn starch | 5 g |
| Magnesium stearate | 5 g |

This formulation is encapsulated in two-piece hard gelatin capsules and dosed at 0.200 g for each capsule.

## Claims

1. A compound which is a 4-substituted 7H-pyrrolo[2,3-d]pyrimidine derivative of formula (I) 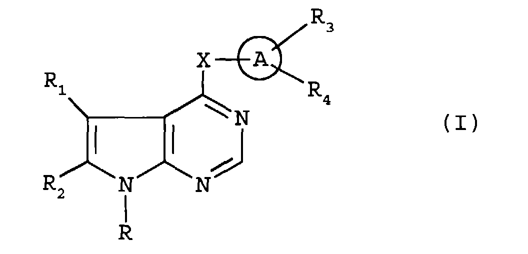 wherein
X is -CH₂-, -NH-(CH₂)ₙ-, -O-(CH₂)ₙ- or -S-(CH₂)ₙ- in which n is zero or 1;
A is a mono- or bicyclic ring chosen from phenyl, pyridine, tetralin, indan, 2-oxindole, quinoline, isoquinoline and indole;
R is hydrogen, C₁-C₄ alkyl or benzyl;
each of R₁ and R₂, which may be the same or different, is independently chosen from hydrogen, halogen, C₁-C₄ alkyl, C₁-C₄ alkoxy, NR₅R₆ in which each of R₅ and R₆ independently is hydrogen or C₁-C₄ alkyl and phenyl unsubstituted or substituted by one to three substituents chosen from halogen, trifluoromethyl, C₁-C₄ alkyl and C₁-C₄ alkoxy;
each of R₃ and R₄, which may be the same or different, is independently hydrogen, C₁-C₄ alkyl, halogen, hydroxy, C₁-C₄ alkoxy, C₁-C₄ alkoxycarbonyl, nitro, cyano or trifluoromethyl;
or a pharmaceutically acceptable salt thereof;
and wherein, when at the same time, X is -NH-(CH₂)ₙ-, -O-(CH₂)ₙ- or -S-(CH₂)ₙ-, R is hydrogen or C₁-C₄ alkyl and A is phenyl, pyridine, quinoline, isoquinoline or indole, then at least one of R₁ and R₂ is other than hydrogen, halogen, C₁-C₄ alkyl, C₁-C₄ alkoxy or NR₅R₆ as defined above;
and wherein, when at the same time, X is -NH-, A is phenyl;
one of R₁ and R₂ is hydrogen, C₁-C₄ alkyl or phenyl unsubstituted or substituted by halogen, trifluoromethyl, C₁-C₄ alkyl or C₁-C₄ alkoxy and the other is C₁-C₄ alkyl or phenyl unsubstituted or substituted by halogen, trifluoromethyl, C₁-C₄ alkyl or C₁-C₄ alkoxy; and R₃ and R₄ are halogen, trifluoromethyl C₁-C₄ alkyl or C₁-C₄ alkoxy;
then R is other than hydrogen.

2. A compound as claimed in claim 1, wherein:
X is -O-, -S-, -CH₂- or -NH-(CH₂)ₙ- in which n is zero or 1;
A is tetralin, indan or 2-oxindole;
R is hydrogen;
R₁ and R₂ are H;
R₃ and R₄ are H or halogen.

3. A compound which is:
4-(2-tetralylthio)-7H-pyrrolo[2,3-d]pyrimidine;
4-(5-indanylamino)-7H-pyrrolo[2,3-d]pyrimidine;
4-(4-indanylamino)-7H-pyrrolo[2,3-d]pyrimidine;
4-(1-tetralylamino)-7H-pyrrolo[2,3-d]pyrimidine;
4-(2-tetralylamino)-7H-pyrrolo[2,3-d]pyrimidine;
4-(5-tetralylamino)-7H-pyrrolo[2,3-d]pyrimidine;
4-(2-oxindol-5-ylamino)-7H-pyrrolo[2,3-d]pyrimidine;
4-(7-bromo-5-indanylamino)-7H-pyrrolo[2,3-d]pyrimidine;
4-(6-bromo-4-indanylamino)-7H-pyrrolo[2,3-d]pyrimidine;
4-(3-bromo-1-tetralylamino)-7H-pyrrolo[2,3-d]pyrimidine;
4-(4-bromo-2-tetralylamino)-7H-pyrrolo[2,3-d]pyrimidine;
4-(5-indanyloxy)-7H-pyrrolo[2,3-d]pyrimidine;
4-(1-tetralyloxy)-7H-pyrrolo[2,3-d]pyrimidine;
4-(2-tetralyloxy)-7H-pyrrolo[2,3-d]pyrimidine;
4-(2-oxindol-5-yloxy)-7H-pyrrolo[2,3-d]pyrimidine;
4-(5-indanylmethyl)-7H-pyrrolo[2,3-d]pyrimidine;
4-(2-tetralylmethyl)-7H-pyrrolo[2,3-d]pyrimidine;
4-(2-oxindol-5-ylmethyl)-7H-pyrrolo[2,3-d]pyrimidine;
4-benzyl-7H-pyrrolo[2,3-d]pyrimidine;
4-(4-pyridylmethyl)-7H-pyrrolo[2,3-d]pyrimidine;
4-(5-indolylmethyl)-7H-pyrrolo[2,3-d]pyrimidine; and
4-(6-quinolylmethyl)-7H-pyrrolo[2,3-d]pyrimidine;
either as single isomer or as a mixture thereof or a pharmaceutically acceptable salt thereof.

4. A pharmaceutical composition comprising a pharmaceutically acceptable carrier and/or diluent, and, as an active principle, a compound as defined in claim 1.

5. A compound as defined in claim 1 for use as an active therapeutic substance.

6. A compound as claimed in claim 5 for use as tyrosine kinase inhibitor.

7. A compound as claimed in claim 5 for use in the control of immune system diseases, in inhibiting the development of atheromatous plaque and restenosis, in the control of angiogenesis, as an anti-metastatic agent, in treating diabetic complications, in the treatment of pathological proliferative conditions or in treating tumors.

8. Products containing a compound as defined in claim 1 and an additional antitumor agent as a combined preparation for simultaneous, separate or sequential use in anti-cancer therapy.

9. Use of a compound of formula (I), or a pharmaceutically acceptable salt thereof, according to claim 1, in the manufacture of a medicament for use as tyrosine kinase inhibitor.

10. A process for the preparation of a compound as defined in claim 1, which process comprises:
a) reacting a compound of formula (II) 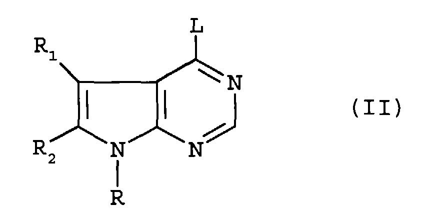 wherein R, R₁ and R₂ are as defined in claim 1 and L is a leaving group, with an amine compound of formula (III) 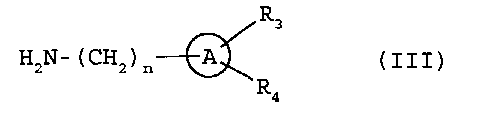 wherein n, A, R₃ and R₄ are as defined in claim 1, thus obtaining a compound of formula (I) in which X is -NH-(CH₂)ₙ-; or
b) reacting a compound of formula (II) as defined above, with an hydroxy compound of formula (IV) 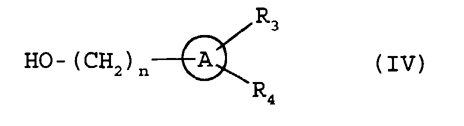 wherein n, A, R₃ and R₄ are as defined in claim 1, thus obtaining a compound of formula (I) in which X is -O-(CH₂)ₙ-; or
c) reacting a compound of formula (II) as defined above, with a thio compound of formula (V) 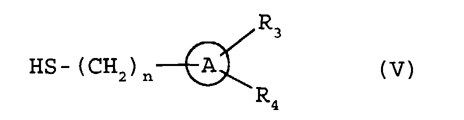 wherein n, A, R₃ and R₄ are as defined in claim 1, thus giving a compound of formula (I) in which X is -S-(CH₂)ₙ-; or
d) hydrolyzing and decarboxylating a compound of formula (VI) 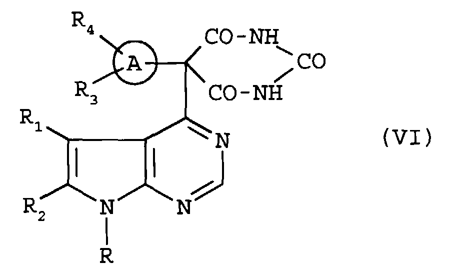 wherein A, R, R₁, R₂, R₃ and R₄ are as defined in claim 1, thus providing compounds of formula (I), wherein X is -CH₂-;
and, if desired, converting a compound of formula (I) into another compound of formula (I), and/or, if desired, converting a compound of formula (I) into a salt thereof, and/or, if desired, converting a salt of a compound of formula (I) into a free compound of formula (I), and/or, if desired, separating a mixture of isomers of a compound of formula (I) into the single isomers.
